# EUROPEAN PATENT APPLICATION

(11) **EP 3 884 926 A1**
(43) Date of publication of application: **29.09.2021**
(21) Application number: 19886809.3
(22) Date of filing: 19.11.2019
(51) Int. Cl.: A61K 8/19, A61Q 17/04, A61Q 19/08

(54) **ULTRAVIOLET RAY-SHIELDING AGENT COMPOSITION ABSORBING RADIATION IN UVA RANGE AND PREPARATION METHOD THEREFOR**

(30) Priority: 19.11.2018 KR 20180142924
(71) Applicant: Soulbrain Co., Ltd., Seongnam-si, Gyeonggi-do 13486 (KR)
(72) Inventor: LEE, Jeong Ho, Seongnam-si Gyeonggi-do 13486 (KR); KIM, Seok Joo, Seongnam-si Gyeonggi-do 13486 (KR)
(74) Representative: M. Zardi & Co S.A.
(86) International application number: PCT/KR2019/015887
(87) International publication number: WO 2020/106038

(57) **Abstract**

Disclosed are a sunscreen composition that absorbs light in the UVA region and a method for preparing the sunscreen composition. Light in the UVA region is light in the wavelength band that affects skin aging and penetrates to the dermis inside the skin. Hence, when the sunscreen composition of the present invention is used, the sunscreen composition absorbs the light and an effect of preventing skin aging is thus obtained.

## Description

### [Technical Field]

The present invention relates to a sunscreen composition that absorbs light in the UVA region and a method for preparing the sunscreen composition.

### [Background Art]

Since the first cosmetic product containing a sunscreen agent was developed in the United States in 1928, the demand for sunscreen agents has steadily increased. Sunscreen agents are intended to prevent skin cancer, sunburn, and photoaging caused by ultraviolet rays. Recently, interest in prevention of photoaging through blocking of ultraviolet rays corresponding to UVA1 and UVA2 wavelengths is increasing for cosmetic purposes. Ultraviolet blocking functions are imparted to most formulations such as BB cream, CC cream, cushion, sun spray, and sun stick in addition to sun cream.

In order to block ultraviolet rays, sunscreen agents are added, and sunscreen agents may be divided into organic sunscreen agents and inorganic sunscreen agents. As organic sunscreen agents, there are typically chemical sunscreen agents that convert light into heat. As inorganic sunscreen agents, there are typically physical sunscreen agents that reflect, scatter, and absorb light. Unlike basic skin care cosmetics, sunscreen is mainly used to attenuate ultraviolet rays on the upper part of the epidermis, that is, the outermost part of the skin. However, in the case of organic sunscreen agents such as avobenzone, the molecular size thereof is small and there is a possibility that the organic sunscreen agent penetrates the skin. Organic sunscreen agents have advantages of having little white turbidness and various absorption wavelengths but may cause skin problems or side effects such as irritation of the eyes when applied around the eyes in the case of sensitive skin. On the other hand, inorganic sunscreen agents are relatively safe and have a favorable blocking effect but are white pigments having high refractive indices, and thus may cause problems such as white turbidness. Due to the recent nature-friendly trend of cosmetic materials, in Korea, the preference for sunscreen products of 'inorganic sunscreen' formulations containing only inorganic sunscreen agents as functional ingredients is high.

Titanium dioxide (TiO₂) and zinc oxide (ZnO) are used as inorganic sunscreen agents but have various disadvantages. First, the energy band gaps of titanium dioxide and zinc oxide are 3.0 eV and 3.2 eV, respectively, and thus titanium dioxide and zinc oxide are advantageous for UVB and UVA2 absorption but cannot absorb UVA1 that is an intermediate wavelength. Second, the refractive indices of titanium dioxide and zinc oxide are as high as 2.7 and 2.2, respectively, and thus white cloudy appearance may be noticeable when the sunscreen is applied to the skin. Third, titanium dioxide and zinc oxide have a great photocatalytic effect to decompose or denature organic materials, particularly coloring matters, under light energy and thus may cause ingredient denaturation of the formulation and pigmentation. In particular, when the photocatalytic effect is great, the surfaces of titanium dioxide and zinc oxide are required to be covered with a second material for safety reasons. In the case of titanium dioxide, aluminum oxide (Al₂O₃) or silicon dioxide (SiO₂) is used to cover 20 parts by weight or more of titanium dioxide. However, when the surface of titanium dioxide is covered with aluminum oxide and silicon dioxide, there may be disadvantages that the powder texture is heavy, the sunscreen is not smoothly applied, and the feel of use is stiff. Hence, it is required to develop a sunscreen composition that can compensate for the above disadvantages.

Accordingly, the present inventors have studied to solve the above problems, found out that a sunscreen composition which can absorb UVA1, suppresses white turbidness by a low refractive index, and is stable because of a low photocatalytic effect can be formed when cerium oxide having the surface modified with a fatty acid is used in the sunscreen composition, and applied for this sunscreen composition (Korean Patent Application No. 10-2017-0142617).

After the above patent application, the present inventors have continued to carry out related studies, found out that light in different wavelength bands in the UVA region is absorbed when the primary particle size and secondary particle size of cerium oxide particles are controlled in the process of related studies, and have thus completed the present invention. Light in the UVA region is light in the wavelength band that affects skin aging and penetrates to the dermis inside the skin. Hence, when the sunscreen composition of the present invention is used, the sunscreen composition absorbs the light and an effect of preventing skin aging is thus obtained.

In this regard, Korean Patent Laid-Open Publication No. 10-2017-0038739 discloses a cosmetic composition for ultraviolet blocking and a method for preparing the same.

### [Summary of Invention]

### [Technical Problem]

The present invention has been devised to solve the above-described problems, and an embodiment of the present invention provides a sunscreen composition that absorbs light in the UVA region.

Another embodiment of the present invention provides a method for preparing the sunscreen composition that absorbs light in the UVA region.

The technical problem to be achieved by the present invention is not limited to the technical problems mentioned above, and other technical problems that are not mentioned will be clearly understood by those skilled in the technical field to which the present invention pertains from the following description.

### [Solution to Problem]

As a technical means for achieving the above-described technical problems, an aspect of the present invention provides a sunscreen composition that absorbs light in a UVA region, the sunscreen composition containing cerium oxide particles that absorb light in a wavelength band of 320 nm to 400 nm.

The primary particle size of cerium oxide (CeO₂) may be 10 to 30 nm, the secondary particle size of cerium oxide (CeO₂) may be 30 to 60 nm, and the ratio of the secondary particle size to the primary particle size may be 1 to 6.

The content of the cerium oxide particles may be 5 parts by weight to 30 parts by weight with respect to 100 parts by weight of the entire sunscreen composition.

Another aspect of the present invention provides a method for preparing cerium oxide particles for ultraviolet blocking that absorbs light in a UVA region, the method including:
preparing a cerium precursor material selected from the group consisting of cerium hydroxide, cerium oxide, cerium carbonate, cerium nitrate, cerium chloride, ammonium cerium nitrate, and combinations thereof;
adding a quaternary ammonium-based material to the cerium precursor material; and
reacting the mixture to which an ammonium-based material is added to obtain cerium oxide (CeO₂) particles.

The quaternary ammonium-based material may include a material selected from the group consisting of ammonium hydroxide, tetramethylammonium hydroxide, tetraethylammonium hydroxide, tetramethylammonium chloride, tetrabutylammonium bromide, tetrabutylammonium fluoride, benzyltrimethylammonium hydroxide, and combinations thereof.

The weight mixing ratio of particles of the precursor material to the quaternary ammonium-based material may be 1: 1 to 3.

The reaction may be performed by a sol-gel method, a supercritical fluid process, a hydrothermal synthesis method, or a coprecipitation method.

The step of obtaining cerium oxide (CeO₂) particles may be carried out by performing a reaction at a temperature of 100°C to 240°C for 18 hours to 30 hours.

The primary particle size of cerium oxide particles obtained in the step of obtaining cerium oxide (CeO₂) particles may be 10 to 30 nm.

The method for preparing cerium oxide particles for ultraviolet blocking that absorbs light in a UVA region may further include removing unreacted materials of the obtained cerium oxide particles after the step of obtaining cerium oxide (CeO₂) particles.

The method for preparing cerium oxide particles for ultraviolet blocking that absorbs light in a UVA region may further include: adding the cerium oxide (CeO₂) particles to an aqueous medium; and milling the aqueous medium after the step of obtaining cerium oxide (CeO₂) particles.

The secondary particle size of cerium oxide particles obtained after the step of milling the aqueous medium may be 30 to 60 nm.

Still another aspect of the present invention provides a method for preparing a sunscreen composition that absorbs light in a UVA region, the method including mixing the aqueous medium with a material selected from the group consisting of silicone oil, a fiber, an emulsifier, a moisturizing agent, a plasticizer, purified water, and combinations thereof after the step of milling the aqueous medium.

### [Advantageous Effects of Invention]

According to an embodiment of the present invention, the sunscreen composition can absorb light in the UVA region of a wavelength band of 320 nm to 400 nm as the primary particle size of the cerium oxide particles is controlled to 10 nm to 30 nm and the secondary particle size thereof is controlled to 30 nm to 60 nm. Light in the UVA region is light in the wavelength band that affects skin aging and penetrates to the dermis inside the skin. Hence, when the sunscreen composition of the present invention is used, the sunscreen composition absorbs the light and an effect of preventing skin aging is thus obtained. Meanwhile, the sunscreen composition may have a high sun protection factor (SPF) and a high PA index and may exhibit excellent dispersion stability since the layer separation thereof does not occur even after a long period of time elapses.

The sunscreen composition has a high dynamic viscosity in the low frequency region and the high frequency region and thus the formulation thereof exhibits excellent emulsification/dispersion phase-stability and the sunscreen composition exhibits excellent application property and thus can be usefully used as a cosmetic composition for ultraviolet blocking.

The sunscreen composition according to an embodiment of the present invention does not cause white cloudy appearance when applied to the skin since the particles have a low light refractive index, and thus can be used as a cosmetic composition for ultraviolet blocking that provides natural impression of color.

The effects of the present invention are not limited to the above effects, and should be understood to include all effects that can be deduced from the configuration of the invention described in the detailed description or claims of the present invention.

### [Brief Description of Drawings]

FIG. 1 is a schematic diagram schematically illustrating that light in the UVA and UVB regions according to an embodiment of the present invention penetrates the skin;
FIG. 2 is a graph illustrating the secondary particle size distribution of cerium oxide particles according to Example of the present invention;
FIG. 3A is a photograph illustrating water-dispersed cerium oxide particles according to Example of the present invention, and FIG. 3B is a photograph illustrating general cerium oxide particles according to Comparative Example of the present invention;
FIG. 4 is a graph illustrating the rate of light absorption depending on the wavelength band by the sunscreen composition according to Experimental Example of the present invention;
FIG. 5A is a graph illustrating the monochromatic protection factor (MPF) of a sunscreen composition according to Comparative Example of the present invention; and
FIG. 5B is a graph illustrating the monochromatic protection factor (MPF) of a sunscreen composition according to Example of the present invention.

### [Description of Embodiments]

Hereinafter, the present invention will be described in more detail. However, the present invention can be implemented in several different forms. The present invention is not limited by the embodiments described herein, and the present invention is only defined by the claims to be described later.

In addition, terms used in the present invention are used only to describe specific embodiments, and are not intended to limit the present invention. Singular expressions include plural expressions unless the context clearly indicates otherwise. In the entire specification of the present invention, "including" a certain component means that other components may be further included rather than excluding other components unless specifically stated to the contrary.

A first aspect of the present application provides a sunscreen composition that absorbs light in a UVA region, the sunscreen composition containing cerium oxide particles that absorb light in a wavelength band of 320 nm to 400 nm.

Hereinafter, the sunscreen composition that absorbs light in the UVA region according to the first aspect of the present application will be described in detail.

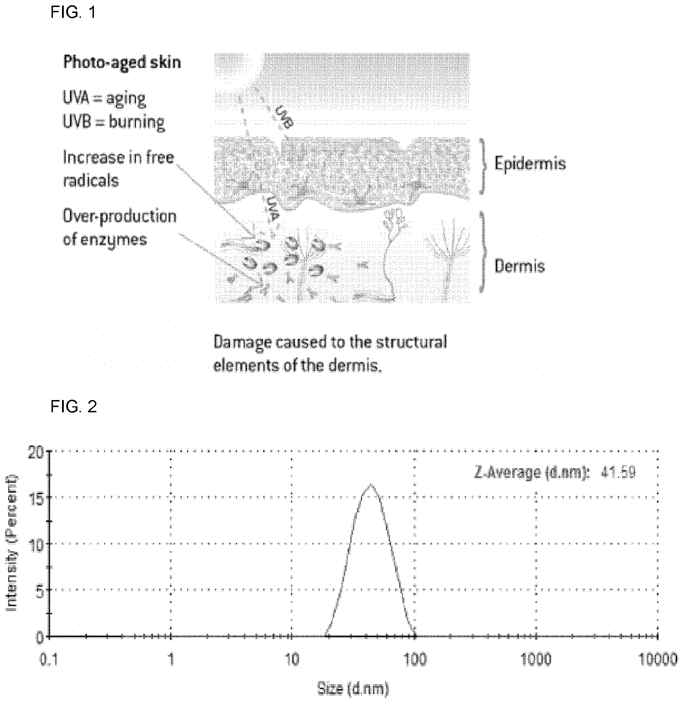

In an embodiment of the present application, light in the UVA region is light in the wavelength band that affects skin aging and penetrates to the dermis inside the skin. Hence, when the sunscreen composition of the present invention is used, the sunscreen composition absorbs the light and an effect of preventing skin aging may be thus obtained. FIG. 1 schematically illustrates that light in the UVA and UVB regions penetrates the skin.

In an embodiment of the present application, light in the UVA region generally refers to light in a wavelength band of about 315 nm to 380 nm, but light in a wavelength band of 320 nm to 400 nm is defined as the UVA region in the present invention. However, it does not completely exclude light in a wavelength band in the ordinary UVA region.

In an embodiment of the present application, the primary particle size of the cerium oxide (CeO₂) particles may be 3 to 50 nm, preferably 5 to 40 nm, more preferably 10 to 30 nm. The secondary particle size of the cerium oxide (CeO₂) particles may be 10 to 90 nm, preferably 20 to 70 nm, more preferably 30 to 60 nm. The ratio of the secondary particle size to the primary particle size may be 0.2 to 30, preferably 0.5 to 14, more preferably 1 to 6.

In an embodiment of the present application, the cerium oxide particles may absorb light in a wavelength band of 320 nm to 400 nm as the primary particle size of cerium oxide is 10 nm to 30 nm and the secondary particle size thereof is 30 nm to 60 nm.

In an embodiment of the present application, the cerium oxide particles may be prepared from a cerium precursor such as a material selected from the group consisting of cerium hydroxide, cerium oxide, cerium carbonate, cerium nitrate, cerium chloride, ammonium cerium nitrate, and combinations thereof. All cerium oxide particles prepared by ordinary cerium oxide preparation methods may be used without particular limitation.

In an embodiment of the present application, the cerium oxide particles may be a cubic, hexagonal, polygonal, spherical, or aggregated spherical shape and may be a mixture of cerium oxide particles having the shapes, but the form and shape of the cerium oxide particles are not limited to the kinds.

In an embodiment of the present application, the content of the cerium oxide particles may be 5 parts by weight to 30 parts by weight, preferably 10 parts by weight to 20 parts by weight, more preferably 20 parts by weight with respect to 100 parts by weight of the entire sunscreen composition. When the content of the cerium oxide particles is less than 5 parts by weight, the content of the cerium oxide particles is too low, the wavelengths in the UVA1 region may not be absorbed, and thus the effects of the sunscreen composition according to the present invention may not be exerted. When the content of the cerium oxide particles exceeds 30 parts by weight, the solid content is too high, the viscosity of the cosmetic may become too high, and thus the application property may be impaired. When the content of the cerium oxide particles is less than 5 parts by weight, it may be difficult to expect the ultraviolet blocking effect.

In an embodiment of the present application, the purity of the powder of the cerium oxide particles may be 90% to 99.99%, preferably 95% to 99.9%, more preferably 98% to 99.9%. When the purity of the powder of the cerium oxide particles is less than 98%, the skin stability of the sunscreen composition may be deteriorated by by-products other than the cerium oxide particles.

In an embodiment of the present application, the zeta potential value of surface charge of the cerium oxide particles may be 10 to 60 mV, preferably 20 to 50 mV, more preferably 30 to 50 mV. When the zeta potential value of surface charge of the cerium oxide particles is less than 10 mV, dispersibility may be weakened by ion repulsion. When the zeta potential value of surface charge of the cerium oxide particles exceeds 60 mV, the cerium oxide particles may be reaggregated because of excessively high charge.

In an embodiment of the present application, the cerium oxide particles may be dispersed in an aqueous medium. The aqueous medium may be purified water or acidic water having a pH of 5 to 7.

In an embodiment of the present application, the content of the aqueous medium may be 1 part by weight to 60 parts by weight, preferably 5 parts by weight to 55 parts by weight, more preferably 10 parts by weight to 50 parts by weight with respect to 100 parts by weight of the sunscreen composition. The content of the aqueous medium may be freely selected within the above-described range depending on the formulation of the sunscreen composition to be prepared.

In an embodiment of the present application, the sunscreen composition may further contain silicone oil, a fiber, an emulsifier, a moisturizing agent, a plasticizer, or purified water.

In an embodiment of the present application, as the silicone oil, dimethicone, cetyl dimethicone, cyclopentasiloxane, cyclohexasiloxane, stearyl dimethicone and the like may be used. The silicone oil may form an oil phase when the cosmetic is emulsified, and plays a role of improving the feel of use.

In an embodiment of the present application, as the fiber, VGL silk and the like may be used. The fiber plays a role of improving the feel of use of the sunscreen composition.

In an embodiment of the present application, as the emulsifier, a PEG silicone emulsifier, a nonionic W/O emulsifier, a cationic emulsifier, an anionic emulsifier and the like may be used. The emulsifier allows each ingredient of the sunscreen composition according to the present invention to be emulsified. The emulsifier plays a role of improving the stability of the formulation by trapping the particles in the emulsion particles in the oil phase.

In an embodiment of the present application, as the moisturizing agent, natural moisturizing factors (NMF) such as polyols including 1,2-hexanediol, glycerin, propylene glycol, butylene glycol, polyethylene glycol, sorbitol or trehalose, amino acids, urea, lactates or PCA-Na, high molecular weight moisturizing agents such as hyaluronates, chondroitin sulfate or hydrolyzed collagen, and the like may be used. The moisturizing agent may increase the moisturizing power of the sunscreen composition according to the present invention and at the same time serve as a preservative.

In an embodiment of the present application, as the plasticizer, DPG (dipropylene glycol) and the like may be used.

In an embodiment of the present application, in addition to the ingredients described above, ingredients usually blended in cosmetic compositions, such as fats and oils, waxes, surfactants, thickeners, coloring matters, cosmetic additives, powders, saccharides, antioxidants, buffers, various extracts, stabilizers, preservatives, and fragrances, may be appropriately blended in the sunscreen composition as long as the effects of the present invention are not impaired.

In an embodiment of the present application, as the fats and oils, vegetable oils such as evening primrose oil, rosehip oil, castor oil, or olive oil, animal oils such as mink oil or squalene, mineral oils such as liquid paraffin or petrolatum, synthetic oil such as silicone oil or isopropyl myristate, and the like may be used.

In an embodiment of the present application, as the waxes, vegetable waxes such as carnauba wax, candelilla wax, or jojoba oil, animal waxes such as beeswax or lanolin, and the like may be used.

In an embodiment of the present application, as the surfactants, an anionic surfactant, a cationic surfactant, an amphoteric surfactant, a nonionic surfactant and the like may be used.

In an embodiment of the present application, as the thickeners, for example, a water-soluble polymer may be used.

In an embodiment of the present application, as the water-soluble polymers, a plant-based (polysaccharide-based) natural polymer such as guar gum, locust bean gum, queen's seed, carrageenan, galactan, gum arabic, tragacanth gum, pectin, mannan, or starch, a microbial (polysaccharide-based) natural polymer such as xanthan gum, dextran, succinoglycan, cadran, or hyaluronic acid, an animal-based (protein-based) natural polymer such as gelatin, casein, albumin, or collagen, a cellulose-based semisynthetic polymer such as methyl cellulose, ethyl cellulose, hydroxyethyl cellulose, hydroxypropyl cellulose, carboxymethyl cellulose, or methyl hydroxypropyl cellulose, a starch-based semisynthetic polymer such as soluble starch, carboxymethyl starch, or methyl starch, an alginic acid-based semisynthetic polymer such as propylene glycol alginate or alginates, semisynthetic polymers of other polysaccharide derivatives, a vinyl-based synthetic polymer such as polyvinyl alcohol, polyvinylpyrrolidone, polyvinyl methyl ether, carboxyvinyl polymer, or sodium polyacrylate, other synthetic polymers such as polyethylene oxide and ethylene oxide or propylene oxide block copolymer, inorganic substances such as bentonite, laponite, fine silicon oxide, and colloidal alumina, and the like may be used.

In an embodiment of the present application, as the coloring matters, for example, a synthetic coloring matters or natural coloring matters may be used. As the synthetic coloring matters, water-soluble/oil-soluble dyes such as FD&C Yellow No. 6 or FD&C Red No. 4, inorganic pigments such as iron oxide or ultramarine, organic pigments such as D&C Red No. 30 or D&C Red No. 36, lakes such as FD&C Yellow No. 6 Al lake, and the like may be used. As the natural coloring matters, a carotenoid-based coloring matter such as β-carotene, β-apo-8-carotenal, lycopene, capsanthin, bixin, crocin, or canthaxanthin, a flavonoid-based coloring matter such as shisonin, lamanin, niacin, carsamin, safrole yellow, rutin, quercetin or cacao pigment, a flavin-based coloring matter such as riboflavin, a quinone-based coloring matter such as laccaic acid, carminic acid (cochineal), kermesic acid, alizarin, cichoriin, arkanine, or nikinochrome, a porphyrin-based coloring matter such as chlorophyll or hemoglobin, a diketone-based coloring matter such as circumin (turmeric), a beta-cyanidin-based coloring matter such as betanin, and the like may be used.

In an embodiment of the present application, as the cosmetic additives, for example, vitamins, plant extracts, or animal extracts may be used. Retinol (vitamin A), tocopherol (vitamin E), ascorbic acid (vitamin C) and the like may be used as the vitamins. As the plant extracts, menthol (mint), azulene (chamomile), allantoin (wheat), caffeine (coffee), licorice extract, cinnamon extract, green tea extract, lavender extract, lemon extract, and the like may be used. As the animal extracts, placenta (bovine placenta), royal jelly (honey bee secretion), snail extract (mucus secretion), and the like may be used.

A second aspect of the present application provides a method for preparing cerium oxide particles for ultraviolet blocking that absorbs light in a UVA region, the method including: preparing a cerium precursor material selected from the group consisting of cerium hydroxide, cerium oxide, cerium carbonate, cerium nitrate, cerium chloride, ammonium cerium nitrate, and combinations thereof; adding a quaternary ammonium-based material to the cerium precursor material; and reacting the mixture to which an ammonium-based material is added to obtain cerium oxide (CeO₂) particles.

Detailed description of the parts overlapping with the first aspect of the present application has been omitted, but the contents described for the first aspect of the present application may be equally applied even if the description thereof is omitted in the second aspect.

Hereinafter, the method for preparing cerium oxide particles for ultraviolet blocking that absorbs light in a UVA region according to the second aspect of the present application will be described in detail.

First, in an embodiment of the present application, the method for preparing cerium oxide particles for ultraviolet blocking includes preparing a cerium precursor material selected from the group consisting of cerium hydroxide, cerium oxide, cerium carbonate, cerium nitrate, cerium chloride, ammonium cerium nitrate, and combinations thereof.

In an embodiment of the present application, a calcium salt may be additionally added other than the cerium precursor such as cerium hydroxide. At this time, the content of the added calcium salt may be 0.1 part by weight to 99.9 parts by weight with respect to 100 parts by weight of the cerium precursor. At this time, the calcium salt may include, for example, a material selected from the group consisting of calcium hydroxide, calcium oxide, calcium carbonate, calcium nitrate, calcium chloride, ammonium calcium nitrate, and combinations thereof. In this case, the obtained particles may be calcium-cerium oxide particles.

In an embodiment of the present application, the obtained cerium oxide particles or calcium-cerium oxide particles may be mixed with zirconia beads at a volume ratio of 1:1. At this time, the mixing may be performed through milling using a bead mill.

Next, in an embodiment of the present application, the method for preparing cerium oxide particles for ultraviolet blocking includes adding a quaternary ammonium-based material to the cerium precursor material.

In an embodiment of the present application, the quaternary ammonium-based material may include a material selected from the group consisting of ammonium hydroxide, tetramethylammonium hydroxide, tetraethylammonium hydroxide, tetramethylammonium chloride, tetrabutylammonium bromide, tetrabutylammonium fluoride, benzyltrimethylammonium hydroxide, and combinations thereof, preferably ammonium hydroxide.

In an embodiment of the present application, the weight mixing ratio of the cerium precursor material to the quaternary ammonium-based material may be 1:1 to 3, preferably 1:1.5 to 2.5. In this case, the pH of the mixture in which the quaternary ammonium-based material is mixed may be 8 to 12, preferably 9 to 11.

Next, in an embodiment of the present application, the method for preparing cerium oxide particles for ultraviolet blocking includes reacting the mixture to which an ammonium-based material is added to obtain cerium oxide (CeO₂) particles.

In an embodiment of the present application, the reaction may be performed by a sol-gel method, a supercritical fluid process, a hydrothermal synthesis method, or a coprecipitation method, preferably by a hydrothermal synthesis method. The sol-gel method, supercritical fluid process, hydrothermal synthesis method, or coprecipitation method is a process generally used to form particles and is a known method, and thus the detailed description thereof will be omitted. However, the reaction may be preferably performed by a hydrothermal synthesis method.

In an embodiment of the present application, the step of obtaining cerium oxide (CeO₂) particles may be performed at a temperature of 100°C to 240°C, preferably at a temperature of 140°C to 220°C, more preferably at a temperature of 160°C to 200°C. The step of obtaining cerium oxide (CeO₂) particles may be performed for 18 hours to 30 hours, preferably for 20 to 28 hours, more preferably for 22 to 26 hours in the above temperature range.

In an embodiment of the present application, the primary particle size of the cerium oxide particles obtained in the step of obtaining cerium oxide (CeO₂) particles may be 3 to 50 nm, preferably 5 to 40 nm, more preferably 10 to 30 nm.

In an embodiment of the present application, the method for preparing cerium oxide particles for ultraviolet blocking may further include removing unreacted materials of the obtained cerium oxide particles after the step of obtaining cerium oxide (CeO₂) particles. The step of removing the unreacted materials of the cerium oxide particles may be performed by a general method, for example, by centrifugation.

In an embodiment of the present application, the method for preparing cerium oxide particles for ultraviolet blocking may further include: adding the cerium oxide (CeO₂) particles to an aqueous medium; and milling the aqueous medium after the step of obtaining cerium oxide (CeO₂) particles.

In an embodiment of the present application, the aqueous medium may be purified water or acidic water having a pH of 5 to 7. At this time, the amount of the cerium oxide (CeO₂) particles added may be 60 parts by weight to 100 parts by weight, preferably 70 parts by weight to 90 parts by weight with respect to 100 parts by weight of the aqueous medium.

In an embodiment of the present application, the secondary particle size of the cerium oxide particles obtained after the step of milling the aqueous medium may be 10 to 90 nm, preferably 20 to 70 nm, more preferably 30 to 60 nm.

In an embodiment of the present application, the milling is not particularly limited as long as a general milling method is used, and may be performed using, for example, a bead mill. The milling may be performed until the secondary particles of cerium oxide have a size of 30 nm to 60 nm.

In an embodiment of the present application, as the primary particle size and secondary particle size of the cerium oxide particles obtained after the step of milling the aqueous medium are 10 to 30 nm and 30 to 60 nm, respectively, the cerium oxide particles for ultraviolet blocking may absorb light in a wavelength band of 320 nm to 400 nm.

A third aspect of the present application provides a method for preparing a sunscreen composition that absorbs light in a UVA region, the method including mixing the aqueous medium with a material selected from the group consisting of silicone oil, a fiber, an emulsifier, a moisturizing agent, a plasticizer, purified water, and combinations thereof after the step of milling the aqueous medium.

Detailed description of the parts overlapping with the first and second aspects of the present application has been omitted, but the contents described for the first and second aspects of the present application may be equally applied even if the description thereof is omitted in the third aspect.

In an embodiment of the present application, the step of mixing the aqueous medium with a material selected from the group consisting of silicone oil, a fiber, an emulsifier, a moisturizing agent, a plasticizer, purified water, and combinations thereof is a step of preparing a formulation that can be used as a cosmetic composition by mixing the cerium oxide particles dispersed in the aqueous medium phase with various mixtures for cosmetic composition preparation. At this time, the kinds of silicone oils, fibers, emulsifiers, moisturizing agents, and plasticizers that can be used are as described in the first aspect of the present application.

### [Examples]

Hereinafter, Examples of the present invention will be described in detail so that those skilled in the technical field to which the present invention pertains can easily implement the present invention. However, the present invention may be implemented in various different forms and is not limited to Examples described herein.

### Preparation Example Preparation of dispersion of water-dispersed cerium oxide particle

### Step 1: Preparation of cerium oxide particle

Particles were grown by hydrothermal reaction that was a chemical synthesis method using cerium nitrate as a cerium oxide precursor in a bottom-up manner. Mixed were 100 parts by weight of the cerium nitrate and 500 parts by weight of deionized water based on 100 parts by weight of cerium nitrate, and 200 parts by weight of aqueous ammonia was added to the mixture while performing stirring to prepare a precursor solution having a pH of 10. The precursor solution was put into a reactor for hydrothermal synthesis and reacted at 180°C for 24 hours to prepare cerium oxide particles. The particles were centrifuged to remove unreacted materials. The cerium oxide particles from which the unreacted materials had been removed had a primary particle size of 14.1 nm.

### Step 2: Preparation of dispersion of water-dispersed cerium oxide particle

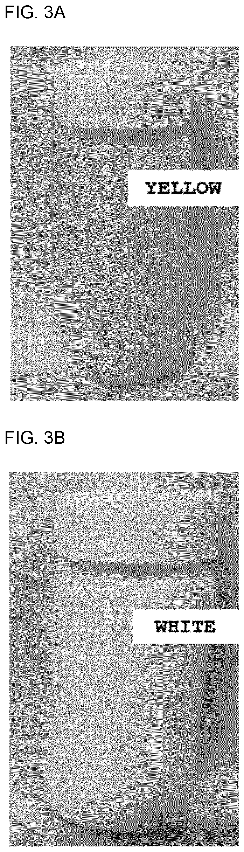

To 2,500 g of deionized water, 50 g of a pH adjusting agent (nitric acid or the like) was added, and then the mixture was stirred. To the prepared solution, 2,000 g of the cerium oxide particles prepared in step 1 was added, and then milling was performed using a bead mill to obtain a dispersion of water-dispersed cerium oxide. At this time, the secondary particle size distribution of the obtained cerium oxide particles is illustrated in FIG. 2, and the average secondary particle size was 41.59 nm. A photograph of the obtained dispersion of water-dispersed cerium oxide particles is illustrated in FIG. 3A.

### Example Preparation of sunscreen composition

The dispersion of water-dispersed cerium oxide particles prepared in Preparation Example (54.55 wt%), silicone oil (DC 245, 10 wt%), a fiber (VGL silk, 10 wt%), a PEG silicone emulsifier (KF 6017, 3 wt%), a nonionic W/O emulsifier (Abil em 90, 2.5 wt%), a moisturizing agent (1,2-hexanediol, 2 wt%), a plasticizer (DPG, 10 wt%), and purified water (7.95 wt%) were mixed together to prepare a sunscreen composition.

### Comparative Example 1 Preparation of sunscreen composition containing general cerium oxide particle

A sunscreen composition containing general cerium oxide particles was prepared by the following method.

### Step 1: Preparation of general cerium oxide particle

One of cerium hydroxide, cerium oxide, cerium carbonate, cerium nitrate, cerium chloride, or ammonium cerium nitrate was put into a crucible and subjected to a heat treatment at 400°C to 1200°C in a heating furnace to obtain cerium oxide particles.

### Step 2: Preparation of dispersion of water-dispersed general cerium oxide particle

To 2,500 g of deionized water, 50 g of a pH adjusting agent (nitric acid or the like) was added, and then the mixture was stirred. To the prepared solution, 2,000 g of the general cerium oxide particles prepared in step 1 was added, and then milling was performed using a bead mill to obtain a dispersion of water-dispersed general cerium oxide. At this time, a photograph of the obtained dispersion of water-dispersed general cerium oxide particles is illustrated in FIG. 3B.

### Step 3: Preparation of sunscreen composition containing general cerium oxide particle

The dispersion of water-dispersed general cerium oxide particles prepared in step 2 (54.55 wt%), silicone oil (DC 245, 10 wt%), a fiber (VGL silk, 10 wt%), a PEG silicone emulsifier (KF 6017, 3 wt%), a nonionic W/O emulsifier (Abil em 90, 2.5 wt%), a moisturizing agent (1,2-hexanediol, 2 wt%), a plasticizer (DPG, 10 wt%), and purified water (7.95 wt%) were mixed together to prepare a sunscreen composition. At this time, the average secondary particle size of the prepared general cerium oxide particles was 150 nm.

### Comparative Example 2 Preparation of sunscreen composition not containing cerium oxide particle

A commonly used sunscreen composition that did not contain cerium oxide particles was prepared.

### Experimental Example 1 Analysis of wavelength region of absorbed light (UV-Vis spectrophotometry)

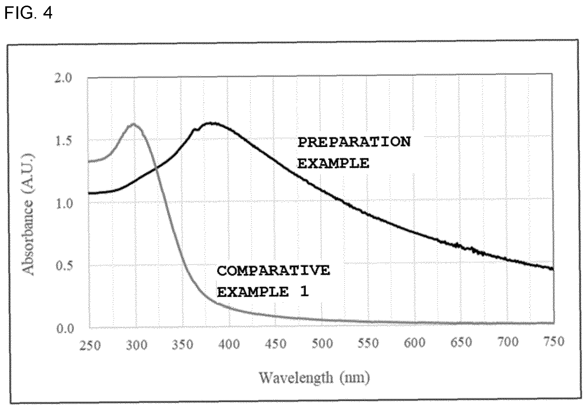

The wavelength regions of light absorbed by the dispersion of water-dispersed cerium oxide particles prepared in Preparation Example and the dispersion containing general cerium oxide particles of Comparative Example 1 were analyzed by UV-Vis spectrophotometry, and the results are illustrated in FIG. 4.

As illustrated in FIG. 4, it has been confirmed that the dispersion of water-dispersed cerium oxide particles prepared in Preparation Example of the present invention absorbs light in a wavelength band of about 320 nm to 400 nm while the dispersion containing general cerium oxide particles of Comparative Example 1 absorbs light in a wavelength band of about 270 nm to 320 nm. Hence, it has been confirmed that the dispersion of water-dispersed cerium oxide particles of the present invention absorbs light in the UVA region.

### Experimental Example 2 Experiment to measure sun protection factor (SPF) and PA index

In order to measure the sun protection factors (SPF) and PA indices of the sunscreen compositions of Comparative Example 2 and Example, SPF and PA were measured using the SPF analyzer 290S of Laser Components, UK, and the results are presented in Table 1 below.

**[Table 1]**

| | Comparative Example 2 | Example |
|---|---|---|
| CeO₂ content (wt%) | 0% | 20% |
| SPF | 10 | 21 |
| PA | (++) | (+++) |

As presented in Table 1 above, it has been confirmed that the sunscreen composition of Example that contains cerium oxide particles having a primary particle size of 14.1 nm and a secondary particle size of 41.59 nm has significantly higher SPF and PA index than the sunscreen composition of Comparative Example 2 that does not contain cerium oxide particles.

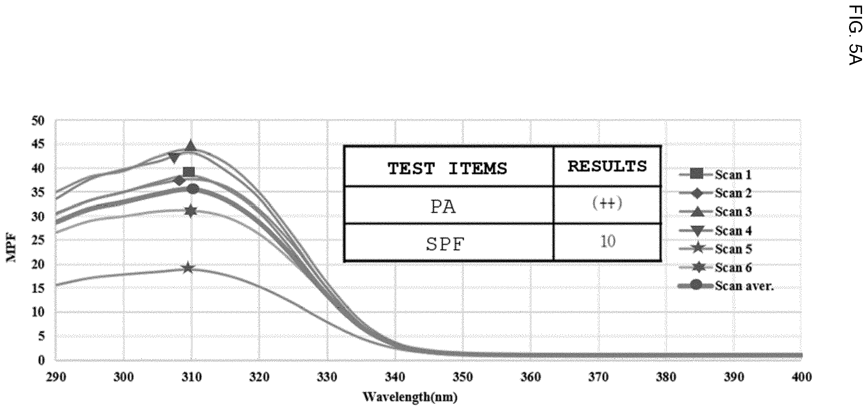

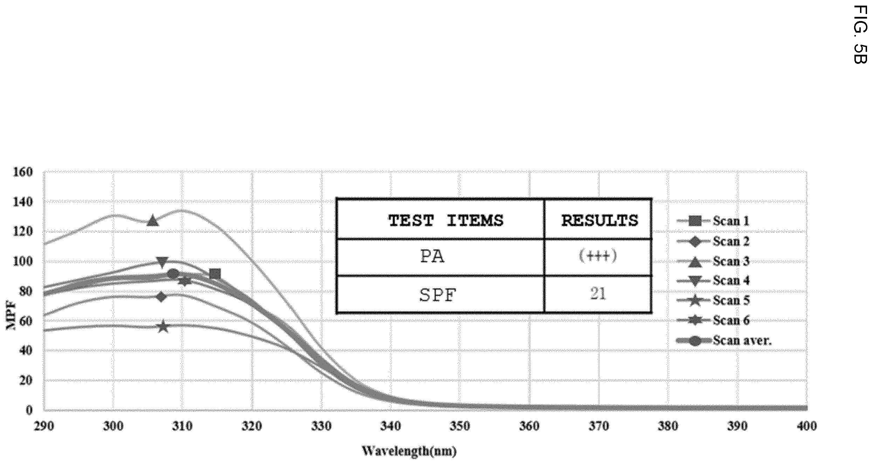

The monochromatic protection factors (single wavelength blocking efficiency, MPF) of the sunscreen compositions of Comparative Example 2 and Example were measured and illustrated in FIGS. 5A and 5B, respectively. As illustrated in FIGS. 5A and 5B, it has been confirmed that the sunscreen composition of Example that contains cerium oxide particles having a primary particle size of 14.1 nm and a secondary particle size of 41.59 nm has a significantly higher MPF than the sunscreen composition of Comparative Example 2 that does not contain cerium oxide particles.

## Claims

1. A sunscreen composition that absorbs light in a UVA region, the sunscreen composition comprising cerium oxide particles that absorb light in a wavelength band of 320 nm to 400 nm.

2. The sunscreen composition that absorbs light in a UVA region according to claim 1, wherein a primary particle size of cerium oxide (CeO₂) is 10 to 30 nm,
a secondary particle size of cerium oxide (CeO₂) is 30 to 60 nm, and
a ratio of the secondary particle size to the primary particle size is 1 to 6.

3. The sunscreen composition that absorbs light in a UVA region according to claim 1, wherein a content of the cerium oxide particles is 5 parts by weight to 30 parts by weight with respect to 100 parts by weight of the entire sunscreen composition.

4. A method for preparing cerium oxide particles for ultraviolet blocking that absorbs light in a UVA region, the method comprising:
preparing a cerium precursor material selected from the group consisting of cerium hydroxide, cerium oxide, cerium carbonate, cerium nitrate, cerium chloride, ammonium cerium nitrate, and combinations thereof;
adding a quaternary ammonium-based material to the cerium precursor material; and
reacting the mixture to which an ammonium-based material is added to obtain cerium oxide (CeO₂) particles.

5. The method for preparing cerium oxide particles for ultraviolet blocking that absorbs light in a UVA region according to claim 4, wherein the quaternary ammonium-based material includes a material selected from the group consisting of ammonium hydroxide, tetramethylammonium hydroxide, tetraethylammonium hydroxide, tetramethylammonium chloride, tetrabutylammonium bromide, tetrabutylammonium fluoride, benzyltrimethylammonium hydroxide, and combinations thereof.

6. The method for preparing cerium oxide particles for ultraviolet blocking that absorbs light in a UVA region according to claim 4, wherein a weight mixing ratio of particles of the precursor material to the quaternary ammonium-based material is 1:1 to 3.

7. The method for preparing cerium oxide particles for ultraviolet blocking that absorbs light in a UVA region according to claim 4, wherein the reaction is performed by a sol-gel method, a supercritical fluid process, a hydrothermal synthesis method, or a coprecipitation method.

8. The method for preparing cerium oxide particles for ultraviolet blocking that absorbs light in a UVA region according to claim 4, wherein the step of obtaining cerium oxide (CeO₂) particles is carried out by performing a reaction at a temperature of 100°C to 240°C for 18 hours to 30 hours.

9. The method for preparing cerium oxide particles for ultraviolet blocking that absorbs light in a UVA region according to claim 4, wherein a primary particle size of cerium oxide particles obtained in the step of obtaining cerium oxide (CeO₂) particles is 10 to 30 nm.

10. The method for preparing cerium oxide particles for ultraviolet blocking that absorbs light in a UVA region according to claim 4, which further comprises removing unreacted materials of the obtained cerium oxide particles after the step of obtaining cerium oxide (CeO₂) particles.

11. The method for preparing cerium oxide particles for ultraviolet blocking that absorbs light in a UVA region according to claim 4, which further comprises:
adding the cerium oxide (CeO₂) particles to an aqueous medium; and
milling the aqueous medium
after the step of obtaining cerium oxide (CeO₂) particles.

12. The method for preparing cerium oxide particles for ultraviolet blocking that absorbs light in a UVA region according to claim 11, wherein a secondary particle size of cerium oxide particles obtained after the step of milling the aqueous medium is 30 to 60 nm.

13. A method for preparing a sunscreen composition that absorbs light in a UVA region, the method comprising mixing the aqueous medium with a material selected from the group consisting of silicone oil, a fiber, an emulsifier, a moisturizing agent, a plasticizer, purified water, and combinations thereof after the step of milling the aqueous medium of claim 11.
